# EUROPEAN PATENT SPECIFICATION

(11) **EP 0 267 402 B1**
(45) Date of publication and mention of the grant of the patent: **07.07.1993**
(21) Application number: 87114085.1
(22) Date of filing: 26.09.1987
(51) Int. Cl.: A61K 37/02, A61K 37/38

(54) **Use of follicle regulatory protein for the production of a therapeutic agent for the treatment of neoplasm of gonadal origin**
Verwendung von Follikelreifungshormon zur Herstellung eines therapeutischen Mittels zur Behandlung von Neoplasmen von gonadalem Ursprung
Utilisation de l'hormone de maturation folliculaire pour la manufacture d'un agent thérapeutique pour le traitement des néoplasmas d'origine gonadale

(30) Priority: 03.10.1986 US 915074
(43) Date of publication of application: 18.05.1988
(73) Proprietor: Decatur-FRP/Partners, Los Angeles California 90064 (US)
(72) Inventor: Morton, Donald L., Pacific Palisades, CA 90272 (US)
(74) Representative: UEXKÜLL & STOLBERG

(56) References cited:
- WO-A-86/04241
- AM. J. OBSTET. GYNECOL., vol. 154, 1986; M.D.TSUTOMU ONO et al., pp. 709-716#
- ARCHIVES OF ANDROLOGY, vol. 16, no. 1, 1986, Hemisphere Publishing Corporation; K.FUJIMORI et al., pp. 25-34#
- OBSTET. GYNECOL., vol. 73, no. 1, January 1989; K.E.RODGERS et al., pp. 66-74#

## Description

### BACKGROUND OF THE INVENTION

The immense complexity of higher organisms requires extremely sensitive and sophisticated systems for regulating and integrating such processes as, for example, growth, regeneration, reproduction, and metabolic rate. Hormones are chemical messengers which play a crucial role in such regulation. Generally synthesized by specialized parts of the body called endocrine glands, they are carried throughout the body in the circulating blood and evoke specific responses in distant targeted tissues and organs. Human reproduction is a prime example of an extremely complex and highly integrated system finely coordinated by a set of interactive hormones. The nature and function of hormones which acts on this system are continually being elucidated.

Recently, a previously unknown hormone termed Follicle Regulatory Protein, or FRP, has been identified. FRP is a protein secreted by the granulosal cells of the ovary which affects such reproductive functions in mammals as the production of sex hormones, growth and development of gametes, and ovulation. Unlike other hormones secreted by the reproductive organs, such as estrogen, which indirectly affect reproductive function by stimulating or inhibiting release of other hormones by endocrine organs such as the pituitary, it appears that FRP directly affects adjacent reproductive cells and tissues. FRP has been demonstrated to inhibit the maturation of ovarian follicles in females and spermatogenesis in males. FRP appears to be primarily responsible for the fact that in certain species, such as man, where single births are the norm, only one egg matures in the ovaries per menstrual or estrus cycle. Thus, FRP appears to have an important role in regulating the activity of normal reproductive cells.

The human reproductive tract is subject to numerous types of malignancies, many of which are difficult to detect and carry a high mortality rate. Two of the most common are ovarian and testicular cancers which result from neoplasms of gonadal cells; together such cancers will account for some 12,000 projected deaths in the United States in 1986.

WO 86/04241 describes a method and composition useful for treating malignant neoplasia and acquired immune deficiency syndrome by administration of a substance characteristic of the tumor or acquired immune deficiency syndrome-afflicted cell in an amount less than the lowest amount necessary to provoke a humoral immune response.

Fujimori et al., 1986, Archives of Andrology, 16: 25 describes the evaluation of the effects of follicle regulatory protein (FRP) on the histology of seminiferous epithelium by its systemic injection into normal dogs. It was found that FRP inhibits spermatogonial transformation of type A into type B.

Surgical removal is currently the only effective treatment for most reproductive malignancies. However, because the tumors are internal, such cancers often remain undetected until the tumor has grown and spread to such an extent that surgical correction is ineffective or impossible. Chemotherapy, while potentially effective in some cases, has limited activity in most patients and is associated with considerable toxicity and undesirable side effects resulting from the wide ranging effects of such treatment.

There thus remains a long-felt need for a therapeutic agent for the treatment of abnormal cell growth of the mammalian reproductive tract which is effective and carries few side effects. The present invention satisfies these needs and provides other related advantages as well.

### SUMMARY OF THE INVENTION

The present invention involves a novel therapeutic agent for the treatment of abnormal cell growths of the mammalian gonadal tissues, such as ovarian and testicular neoplasms. The agent is effective in lessening the growth of cancer cells where the malignancy is detected early and, in addition, may be used post-operatively to prevent recurrence or spread of the tumor. The agent is of particular utility in that it has few side effects and avoids the use of potentially toxic chemotherapeutic agents.

According to one aspect of the invention, there is provided the use of an agent for the treatment of neoplasms of gonadal origin comprising FRP in therapeutically effective doses capable of slowing down the growth of cancer cells. Such agents are administered intravenously, interperitoneally, or intramuscularly. The administration may be repeated at intervals. The FRP-containing therapeutic agent may also be administered as an adjunct to surgical therapy during the post-operative period to inhibit the growth of any neoplastic cells remaining in the body after surgery.

It will be appreciated from the foregoing that the present invention provides a novel therapeutic agent for the treatment of malignancies which have been heretofore difficult to control or eliminate. Other features and advantages of the present invention will become apparent from the following more detailed description, which illustrates, by way of example, the principles of the invention.

### DETAILED DESCRIPTION OF THE INVENTION

FRP, a hormone produced by the granulosal cells of the ovary, inhibits normal maturation of ovarian follicles and spermatogenesis. As chemical messengers, hormones exert their effect on appropriate tissues by binding to specific receptor sites located on the surfaces of targeted cells. Such binding then sets into motion a complex cycle of molecular events resulting in an evoked response by the targeted tissue. Normally, only a limited number of cell types will express a receptor to any one hormone, thus resulting in the specificity of the hormonal effect. Moreover, many hormones produce a cascade of effects by stimulating or inhibiting cells of other endocrine glands, thus quantitatively or qualitatively changing the composition of hormones in the circulating blood.

FRP exerts a direct effect on certain normal cells of the female reproductive systems, such as the granulosal cells of the ovary. Unexpectedly, however, administration of FRP has been discovered to have an inhibitory effect on neoplastic growth and maturation of other reproductive cell types. Thus, according to the present invention, FRP is used for the production of a therapeutic treatment for the effective and safe lessening of the tumor burden in malignancies of the ovaries and testes. Moreover, as an adjunct to operative removal of the tumor mass, the FRP-containing therapeutic agent inhibits growth of any remaining neoplastic cells.

FRP, which may be recovered from the follicular fluid of certain mammals, such as pigs, mice, monkeys, and humans, or may be chemically synthesized, is diluted with a physiologically acceptable solution, such as phosphate buffered saline (PBS). The solution is then administered in therapeutically effective doses interperitoneally, intramuscularly, or, preferably, intravenously. Treatments are repeated at intervals as necessary to effect a retardation of neoplastic cell growth in cases where surgical removal of the tumor is not indicated. Alternatively, such an FRP solution is administered post-operatively at regular intervals to inhibit regrowth of any remaining cancerous cells.

For example patients exhibiting recurrent ovarian cancer and those demonstrating metastasis of ovarian cancer beyond the abdominal cavity can be treated with FRP to retard tumor cell growth. FRP can be prepared from mammalian follicular fluid according to the method of Example I or ovarian venous blood according to the method of Example VIII. Alternatively, it can be chemically synthesized or produced by recombinant DNA technology utilizing methods well known in the art. From the lyophilized state, FRP is dissolved in an appropriate physiologically acceptable carrier such as PBS.

Patients can be treated by intramuscular injection or continuous intravenous infusion of FRP in an appropriate physiologically acceptable solution. The appropriate dosage will be determined by assaying the effect of FRP at varying levels upon the growth of samples of malignant gonadal tissues according to the chemosensitivity assay method detailed in Example V. An appropriate dose can be administered which is effective to provide the serum levels of FRP which are found to cause maximum inhibition of neoplastic growth in the chemosensitivity assay. The proper dose, which varies according to such factors as the particular type of neoplasm, the renal function of the patient, menopausal state of the patient, general condition of health of the patients, and species of origin of the FRP, generally ranges between about 50 and 500 mg FRP/m² of body surface/day.

After a thirty-day period, the effect of FRP on the growth rate of the metastatic measurable tumor can be evaluated. If the tumor is stable in size or has decreased in size, the therapeutic regime is continued until there is evidence of tumor progression based upon standard clinical criteria utilizing physical examination, diagnostic x-rays, radionucleotide scans and other appropriate diagnostic tests.

In another protocol, patients who have been previously treated by surgical reduction of their tumor burden and have no gross evidence of tumor remaining following surgery can be placed on a maintenance program of continuous daily intramuscular injections of FRP at a dose of 50 to 500 mg/m² of body surface/day. This dose of FRP is continued for up to about two years, or until there is evidence of tumor regrowth and progression, as detected by standard clinical criteria.

### EXAMPLE I

### PREPARATION OF FOLLICLE REGULATORY PROTEIN (FRP)

FRP was prepared from porcine follicular fluid according to the method of Ono, et al., 1986, Am. J. Obstet. Gynecol. 154:709. Briefly, porcine follicular fluid was repeatedly extracted with ammonium sulfate. The 0% to 35% saturated ammonium sulfate extract was percolated through a gel column containing dye matrix gel orange A equilibrated with the buffer consisting of 0.02 mol/liter Tris pH 7.5. Bound fractions were eluted with the equilibration buffer plus 0.5 mol/liter potassium chloride. Fractions containing activity as determined by aromatase inhibition, as detailed in Ono, supra, were pooled, dialyzed against distilled water and lyophilized.

The material was further purified through anion-exchange chromatography using a Mono Q Column. Fractions containing FRP were injected as a Mono P hydrogen ion exchange column and eluted fractions exhibiting FRP activity were further purified by preparative scale gel-exclusion chromatography on an Spherogel TSK G30000SWG Column fitted to a Waters high performance liquid chromatograph. Resulting samples were reduced by ultrafiltration (Amicon Diaflow system with PM 10 membranes). Samples with FRP activity were used in subsequent examples.

FRP isolated and purified in this way was found to be approximately 5% pure. Thus 100 mg of solution contains approximately 5 mg of pure FRP. Unless otherwise indicated, for the purposes of the following examples, amounts of FRP used will be provided in terms of a solution having a purity of 5%. However, this amount can be easily converted to an approximate equivalent of pure FRP by multiplying by 0.05. Where used, the term "pure FRP" shall means the equivalent of essentially 100% pure FRP.

The FRP so isolated and purified was lyophilized and stored in glass vials at room temperature. When ready for use, the lyophilized FRP was dissolved in Roswell Park Memorial Institute Tissue Culture Media Number 1640 (hereinafter "RPMI 1640"), in a concentration of 0.5 to 400 µg/ml.

### EXAMPLE II

### PREPARATION OF TISSUE SAMPLES

Human tumor tissues were obtained from individuals surgically treated for ovarian or testicular cancer. Viable-appearing, non-necrotic tissue was selected from surgically resected tumors, and adipose and connective tissue removed. The tissue was minced into pieces less than two millimeters in diameter in the presence of RPMI 1640 containing 15% heat-inactivated fetal calf serum (Flow Laboratories, McLean, VA).

### EXAMPLE III

### PREPARATION OF SINGLE CELL SUSPENSIONS

Ten to 20 ml of enzyme media consisting of RPMI 1640 containing 0.01% DNAase (500 Kunitz units per ml; Sigma Chemical Company St. Louis, MO) and 0.14% collagenase Type I (Sigma) were added per gram of tumor fragment. The mixture was stirred for 90 minutes at 37° C in the presence of 5% CO₂. After enzymatic digestion, the free cells were decanted through 12 layers of sterile gauze and centrifuged at 200 x g for 10 minutes. The supernatant was removed, cells were resuspended in RPMI 1640 and viability determined by trypan blue exclusion. The cells were centrifuged again and the cell pellet resuspended in RPMI 1640 containing 15% fetal calf serum and 10% dimethyl-sulphoxide (DMSO) in which the cells were placed in a screw cap vial in a program freezer and cooled at one degree per minute for storage at liquid nitrogen temperatures.

### EXAMPLE IV

### FRP TREATMENT OF CELL SUSPENSION

Ampules of the tumor cells were removed from the liquid nitrogen freezer, thawed in a 37° C waterbath, placed in a centrifuged tube, and spun at 200 x g for 10 minutes. The supernatant was removed and the cells were resuspended in RPMI 1640 containing 15% fetal calf serum, after which they were placed in an incubator at 37° C containing 5% CO₂ and allowed to recover overnight. The next day, the cells were washed four times in RPMI 1640 with 300 mg glutamine/l.

Cell viability was determined by trypan blue exclusion and using 0.05% trypan blue in phosphate buffered saline. Eighty thousand cells were placed in test tubes and centrifuged at 200 x g for 10 minutes. The serum-free media was removed and each sample was then resuspended in 100 µl (lambda) of RPMI 1640 containing the particular concentration of FRP being tested. The cells and FRP mixture were incubated for two hours at 37° C.

### EXAMPLE V

### CHEMOSENSITIVITY ASSAY

Cells were cultured on an underlayer of 0.5% agarose (SeaPlaque, FMS Corporation, Rockland, ME), which was prepared by mixing 3.5 ml molten 3% agarose with 16.5 ml RPMI 1640 supplemented with 15% heat-inactivated fetal calf serum, 2 mM L-glutamine (GIBCO, (Grand Island Biological Company) Grand Island, NY), 100 units/ml penicillin, 100 µg/ml streptomycin, and 1.25 µg/ml fungizone. One-half ml of this agarose mixture was added to each 16 x 18 mm well of 24-well plates (Costar 3524, Costar, Cambridge, MA), and the plates were refrigerated for 10 minutes at 4° C. Cells were suspended in 0.6% agarose in RPMI 1640 supplemented with antibiotics and 15% fetal calf serum as above. One-half ml of the cell suspension was added to each of three underlayers at a final concentration of 80,000 cells/well.

FRP purified as described in Example I was added to samples as an overlayer in 300 µl of RPMI 1640, in concentrations of 0.5 to 400 µg/well. Controls were prepared as above with FRP omitted from the overlayer. After 48 hours incubation at 37° C, 1.85 · 10⁵ Bq (5 µCi) of tritiated thymidine (specific activity 7,4 · 10¹⁰ Bq (2.0 Ci)/mM, New England Nuclear, Boston, MA) were layered over each well, and the plates were returned to the incubator for an additional 48 hours. Incorporation of thymidine was terminated by transferring the agarose layers from each well to 15 ml centrifuge tubes (No. C3051-870, Beral Scientific, Arleta, CA) and boiling the tubes for 15 minutes in a waterbath. The volume was brought up to 13 ml with phosphate buffered saline (PBS) (GIBCO) and the tubes were centrifuged; pellets were washed with PBS and then dissolved in 3 ml of 0.85N KOH for one hour at 80° C. Tubes were cooled on ice to below 4° C and the hydrolysates were precipitated by the addition of 30 µl of 1% human serum albumin (Cutter Biological, Berkeley, CA) and 2.4 ml of ice-cold 30% trichloroacetic acid (TCA). After overnight storage at 4° C, the precipitates were collected by centrifugation. The pellets were washed with 4% trichloroacetic acid, dissolved in 0.3 ml of 0.075N KOH, and transferred to scintillation vials containing 5 ml Liquiscint (National Diagnostics, Somerville, NJ). Radioactivity in each vial was measured in a Beckman liquid scintillation counter (LS230, Beckman Instruments, Irvine, CA). An assay was considered evaluable if the average count of the untreated (no FRP) controls was greater than 300 cpm. Cell suspensions of 5 adenocarcinomas of the ovary, 1 seminoma and 1 melanoma prepared as in Examples II and III were treated and assayed as in Examples IV and V. Inhibition of a cell growth was measured by comparing the number of counts in the tubes exposed to FRP to the controlled tubes exposed to RPMI 1640 alone.

### EXAMPLE VI

### EFFECT OF FRP TREATMENT ON OVARIAN ADENOCARCINOMA CELLS

Table I shows the results of chemosensitive assays on the adenocarcinomas and melanomas at varying concentrations of FRP. FRP was found to cause inhibition of cell growth as measured by inhibition of thymidine uptake against four out of five ovarian adenocarcinomas tested. In duplicate experiments, activity was found at 25 µg per dose against adenocarcinoma Sample Nos. 1, 2, 3, and 4, at 5 µg per dose activity was seen against adenocarcinoma #1 and minor activity against adenocarcinomas Sample No. 4. No significant activity was found at 0.5 mg against any of the ovarian adenocarcinomas. When FRP was tested at higher concentrations of 40 to 400 µg, no inhibition was seen at higher concentrations of FRP shown on Table I except it was still active at 40 µg per ml.

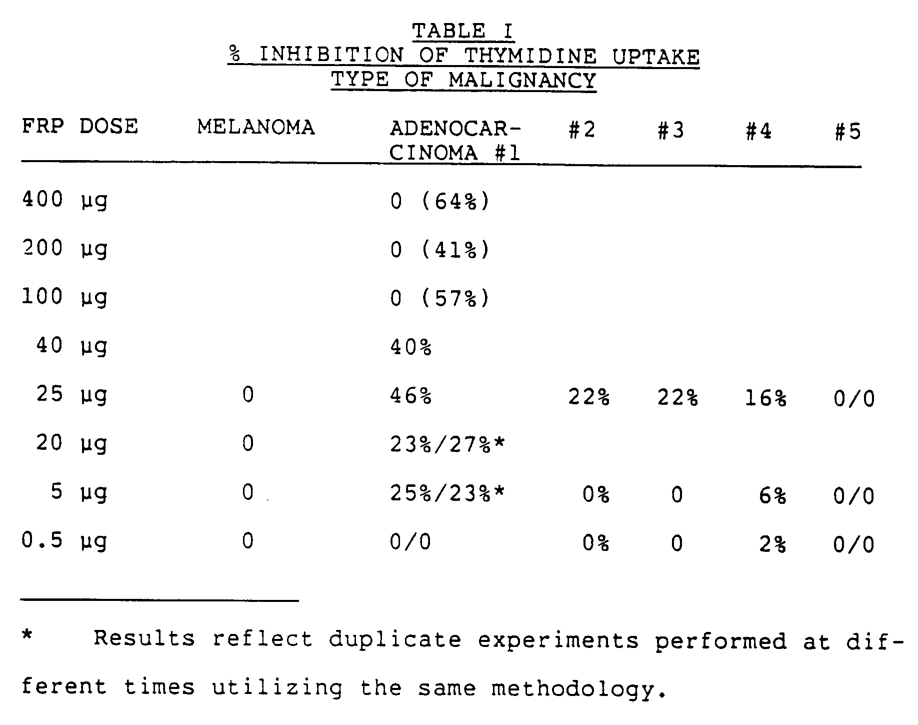

### EXAMPLE VII

### EFFECT OF FRP TREATMENT ON SEMINOMA

FRP was tested against testicular cancer (seminoma) at a dose of 25 µg, 5 µg, and 0.5 µg per ml. Human testicular cancer was very sensitive as seen in Table II to FRP. Fifty-one percent inhibition was observed at the dose of 25 µg FRP per ml. Significant inhibition was also observed at a concentration of 0.5 µg per ml. Inhibition curves for testicular cancer suggest that there is a portion of the testicular carcinoma population of cells that are very sensitive to inhibition by FRP.

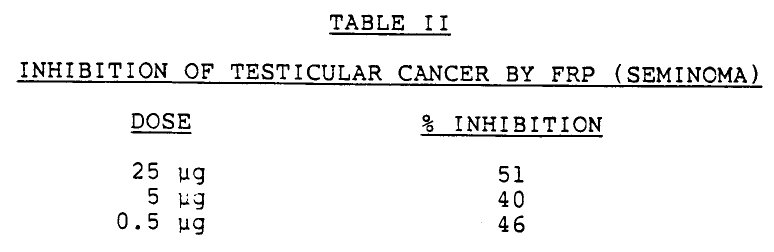

### EXAMPLE VIII

### PREPARATION OF FRP FROM HUMAN OVARIAN VENOUS BLOOD

Ovarian venous blood (5 ml) was collected from six women (aged 26, 28, 31, 36, 37 and 41 years) undergoing laparotomy for indications not related to ovarian dysfunction on days 12-14 after the onset of the last menstrual period. Patients 1-3 maintained regular menstrual cycles, while patients 4-6 were anovulatory, as evidenced by oligomenorrhea and a lack of large antral follicles in the ovarian cortex. A 25-gauge needle was inserted into the venous drainage within the infundibulo-pelvic ligament, and free-flowing blood was aspirated. In addition, the locus of the preovulatory follicle was determined by direct visual inspection. Peripheral serum (10 ml) was collected concurrently from an antecubital vein. Serum was separated by centrifugation (800 x g for fifteen minutes) of the clotted specimen and stored frozen (-35°C) until fractionation. Concentrations of 17β-estradiol in ovarian (1340, 886, and 470 pg/ml) and peripheral (248, 261, and 201 pg/ml) venous samples collected from patients 1, 2 and 3, respectively, were consistent with the preovulatory 17β-estradiol levels reported for normal women. In addition, comparable samples from anovulatory patients 4-6 contained low levels of 17β-estradiol in both ovarian ( 200 pg/ml) and peripheral (50 pg/ml) sera. Slowly thawed serum was fractionated by the dropwise addition of an equal volume of saturated ammonium sulfate under persistent agitation at 4°C. After twelve hours, the precipitate was resuspended (2:1, vol/vol) with 10% ammonium sulfate. After twelve hours of additional agitation and centriguation (3000 x g for 30 minutes), the supernatant was dialyzed with 10,000 molecular weight exclusion membranes against Dulbecco's phosphate-buffered saline (PBS1 0.025 M; pH 6.8) for 16 hours. the retentate was passed through a Concanavalin A-linked Sepharose 4B (Con A ) column (5 ml; Pharmacia, Piscataway, New Jersey) which was washed with 5 vol 0.5 M NaCl, pH 7.4, then further eluted with 0.5 M α-methyl-D-mannoside in PBS at a flow rate of 20 ml/h at 4°C.

Additional fractionations were performed where indicated on a Sephadex G-25 (superfine) column (1.6 x 50 cm; Vo = 60 ml; 5 ml/h; 4°C) with PBS. All Sephadex molecular weight seiving was performed using a reverse flow technique. Both Sephadex G-50 and G-25 were prepared according to the instructions of the manufacturer and equilibrated in the elution buffer. To increase resolution, the smallest of the Sephadex beads were removed by direct pipetting of the surface before degassing and column packing (98.1 Pa (10 mm H₂O)). Elution profiles were determined using an ISCO absorbance meter at 254 nm.

The activity was assessed in twenty-three-day-old Sprague-Dawley rats (45-55 g) 2 days after hypophysectomy which were kept at 25°C with intervals of fourteen hours of light and ten hours of darkness. Animals were caged in groups of three and given rat chow and water ad libitum. Silastic implants containing DES (diethylstilbestrol) were prepared as follows. Ten grams of Silastic (TM) polymer were mixed with 3 g DES for thirty minutes at 16°C; thereafter, four drops of stannous octoate catalyst were added, with an additional ten minutes of mixing. The material was passed through a Luer-lock syringe (id, 1 mm) into a steaming (95°C) 0.9% NaCl water bath and annealed for two hours. DES-containing Silastic implants (1 x 5 mm) were inserted so in the hypophysectomy incision forty-eight hours before assay. The assay design consisted of three rats at each dose of reference preparation and unknown. Forty-eight hours after hypophysectomy, animals were given either varying concentrations of gonadotropins (LH:FSH, 1:1) dissolved in 0.15 M NaCl with 1% bovine serum albumin and/or equal volumes of test fractions in two divided daily doses. Twenty-four hours after the initial injection, animals were sacrificed by decapitation, and ovaries were removed, trimmed and weighed on a Roller-Smith balance. Rat trunk serum 17β-estradiol determinations were performed by methods described in Goeblesmann et al., in Leprow et al (Eds.) Vasectomy: Immunological and Pathologic Effects in Animals and Man, Academic Press, New York, p. 165. Control determination of chromatography fractions containing inhibitory activity was performed by heating (56°C; for thirty minutes) or trypsin digestion (20 mg/100 ml) of representative samples for 4 hours.

The absorbency, at 254 nm, of the Sephadex G-50 fractions from a preovulatory ovarian venous sample of patient 1 to the bioassay results of the sample was determined by rat ovarian weight and serum 17β-estradiol concentrations. An initial peak rose to 28 absorbency units, followed by a gradual downward slope, with the emergence of a smaller second peak (Vₑ/Vₒ=1.42-1.55). When these eluents were tested in the bioassays, the combined rat ovarian weights ranged from 57-100 mg, and rat serum 17β-estradiol levels ranged from 70-230 pg/ml throughout the initial fractions. Thereafter, fractions with a Vₑ/Vₒ of 1.42-1./55 corresponded to an inhibition of hMG-induced ovarian stimulation in the bioassay, as evidenced by a decrease in ovarian weight (59 ± 0.5 mg) and a significant (P<0.01, by paired t tests) decrease in serum 17β-estradiol to levels less than 25 pg/ml. As a consequence, these fractions were pooled and processed for dose response activity. Peripheral and ovarian venous blood collected from the ovary contralateral to the site of ovulation in patient 1 demonstrated similar G-50 elution profiles (data now shown). However, when representative fractions were tested by bioassay, no reduction in ovarian weight or serum 17β-estradiol was found. Further, ovarian venous blood preparations from the anovulatory patients also failed to suppress the response of the ovaries to hMG stimulation. However, ovarian venous sera from the ovulatory ovary of patients 2 and 3 had a similar Sephadex G-50 elution profile. Fractions with a Vₑ/Vₒ=1.48-1.60 suppressed the response of rat ovarian weight (57.4 ± 2.1 vs. 81.2 ± 4.5 mg; P<0.05) and serum 17β-estradiol concentrations ( 25 vs. 68-120 pg/ml; P<0.01) to hMG stimulation. When active fractions from the G-50 eluents of patients 1 - 3 were heated or trypsin digested, they lost their ability to suppress ovarian weight or 17β-estradiol secretion in response to hMG stimulation.

The dose-response curve of ovarian weight suppression in the DES-treated rat ovaries by active Sephadex G-50 fractions (Vₑ/Vₒ=1.42-1.55) was derived from patient 1. Analysis of the rat ovarian weight and serum 17β-estradiol concentrations revealed a linear dose-response pattern. When these same fractions were treated with heat or trypsin, no suppression of ovarian weight was present.

The isoelectric point of active fractions eluted from the Sephadex G-50 column was estimated by ampholyte displacement chromatography. Pooled aliquots (1 ml) of active fractions were layered on a Polybuffer Exchanger 94 (25 ml; equilibrated to pH 7.4 with 0.025 M Imidazole HCl) column (0.6 x 30 cm). Fractions were eluted with Pharmalyte⁽™⁾ polybuffer⁽™⁾ 74-HCl adjsuted to Ph 4.0 with HCl 1 N at 10 ml/h at 4°C. Fractions that eluted at a pH greater than 7.4 were collected and rechromatographed in the same Pharmalyte column reequilibrated to pH 0.4 with ethanolamine 0.025 M and eluted with Polybuffer 96 adjusted to pH 6.0 with 1 N KOH.

The active Sephadex G-25 fractions (Vₑ/Vₒ=1.20-1.25) pooled and developed by ampholyte displacement chromatograms from patient 1 for elution ranges pH 9-4 was compiled. The bioassay results from ovarian weight and serum 17β-estradiol concentrations from representative fractions suggested that the isoelectric point of the active Sephadex G-50 eluents from patient 1 were between pH 6.2-6.5.

Fractions corresponding to a pH of 6.1-6.5 manifest inhibitory activity in excess of 50% tested in the bioassay with hypophysectomized, twenty-three-day-old, DES-treated rat ovaries receiving hMG therapy (ovarian weight). Ampholyte displacement chromatography of the fractions with follicle-inhibiting activity was obtained on Sephadex G-25 gel filtration fractions with V/Vₒ=1.20-1.25.

The Kₐᵥ values for the molecular weight standards and active Sephadex G-50 fractions from patients 1-3 were derived. Estimations of molecular weight ranged from 14,000-16,500 for patient 1, from 14,000-17,000 for patient 2, and from 16,000-18,000 for patient 3.

Thus, it is seen that at least one protein which suppresses the follicle response to gonadotropins is secreted by the preovulatory ovary. Specifically, a heat- and trypsin-labile substance in secreted directly into the venous drainage from the ovary containing the dominant follicle which suppresses the follicular response to gonadotropins. That this protein is not secreted in large amounts by anovulatory ovaries was evidenced by the failure of the bioassay to detect inhibitory activity in the venous drainage of the contralateral ovary of patients 1-3 as well as the ovarian venous effluents from three anovulatory women. This potential intra- and/or interovarian regulator of folliculogenesis mediates dominance of the preovulatory follicle by an active process, such that after the selection of the dominant follicle, the gonadotropin responsivity of other follicles on the same and contralateral ovaries is suppressed.

## Claims

1. Use of Follicle Regulatory Protein for the production of a therapeutic agent for the treatment of neoplasm of gonadal origin.

2. Use according to claim 1 characterized in that the therapeutic agent is formulated in a dosage unit allowing to administer 50-500 mg/m² of pure Follicle Regulatory Protein per patient body surface per day.

3. Use according to claim 2 characterized in that the formulation of the dose unit is determined through a chemosensitivity assay utilizing cells derived from the patient's neoplasm of gonodal origin.

4. Use according to one of the claims 1 to 3 for the treatment of adenocarcinoma of the ovary.

5. Use according to claims 1 to 3 for the treatment of seminoma of the testes.

6. Use according to claims 1 to 5 wherein the therapeutic agent is provided for intramuscular injection.

7. Use according to claims 1 to 5 wherein the therapeutic agent is provided for intravenous infusion.

## Patentansprüche

1. Verwendung des follikelregulierenden Proteins zur Herstellung eines therapeutischen Mittels zur Behandlung von Neoplasmen gonadalen Ursprungs.

2. Verwendung nach Anspruch 1, **dadurch gekennzeichnet,** daß das therapeutische Mittel in einer Dosiseinheit formuliert ist, welche es erlaubt, 50 bis 500 mg des reinen follikelregulierenden Proteins pro m² Körperoberfläche eines Patienten pro Tag zu verabreichen.

3. Verwendung nach Anspruch 2, **dadurch gekennzeichnet,** daß die Formulierung der Dosiseinheit mittels eines Chemosensitivitäts-Assays bestimmt ist, bei dem Zellen verwendet werden, die sich aus dem Neoplasma gonadalen Ursprungs des Patienten ableiten.

4. Verwendung nach einem der Ansprüche 1 bis 3 zur Behandlung des ovariellen Adenokarzinoms.

5. Verwendung nach den Ansprüchen 1 bis 3 zur Behandlung des Seminoms der Hoden.

6. Verwendung nach den Ansprüchen 1 bis 5, bei der das therapeutische Mittel zur intramuskulären Injektion bereitgestellt wird.

7. Verwendung nach den Ansprüchen 1 bis 5, bei der das therapeutische Mittel zur intravenösen Infusion bereitgestellt wird.

## Revendications

1. Utilisation de la Protéine Régulatrice Folliculaire pour la production d'un agent thérapeutique pour le traitement d'un néoplasme d'origine gonadique.

2. Utilisation selon la revendication 1, caractérisée en ce que l'agent thérapeutique est formulé en une dose unitaire permettant l'administration de 50-500 mg/m² de la Protéine Régulatrice Folliculaire pure par surface de corps du patient par jour.

3. Utilisation selon la revendication 2, caractérisée en ce que la formule de la dose unitaire est déterminée par une analyse de chimiosensibilité en utilisant des cellules dérivées du néoplasme d'origine gonadique du patient.

4. Utilisation selon l'une des revendications 1 à 3, pour le traitement de l'adénocarcinome de l'ovaire.

5. Utilisation selon les revendications 1 à 3 pour le traitement du séminome des testicules.

6. Utilisation selon les revendications 1 à 5, où l'agent thérapeutique est prévu pour injection intramusculaire.

7. Utilisation selon les revendications 1 à 5, où l'agent thérapeutique est prévu pour infusion intraveineuse.
